(19) Europäisches Patentamt
European Patent Office
Office européen des brevets

(11) Numéro de publication: **0 026 168**
**B1**

# (12) FASCICULE DE BREVET EUROPEEN

(45) Date de publication du fascicule du brevet: **16.03.83**

(51) Int. Cl.³: **C 07 D 215/22**, A 61 K 31/47

(21) Numéro de dépôt: **80870039.7**

(22) Date de dépôt: **18.09.80**

## (54) Nouveaux dérivés de décahydroquinoléinol, leur procédé de préparation ainsi que leurs applications en thérapeutique.

(30) Priorité: **19.09.79 FR 7923289**

(43) Date de publication de la demande: **01.04.81 Bulletin 81/13**

(45) Mention de la délivrance du brevet: **16.03.83 Bulletin 83/11**

(84) Etats contractants désignés: **BE CH DE FR IT LI LU NL SE**

(56) Documents cités:
**FR-A-2160939**

**«European Journal of Medicinal Chemistry — Chimica Terapeutica», vol. 11, N° 4, Chatenay-Malabry, France, M. Prost *et al.*: «Dérivés de la décahydroquinoléine VI(1). Hydroxy-4 décahydroquinoléines et dérivés N-substitués», pages 337-342.**

(73) Titulaire: **SANOFI, société anonyme, 40, Avenue George V, F-75008 Paris (FR)**

(72) Inventeur: **Prost, Maurice, Avenue des Camélias, 48, B-1150 Bruxelles (BE)**
Inventeur: **de Claviere, Michel, Heyzellaan, 28, B-1800 Vilvoorde (BE)**

(74) Mandataire: **Cauchie, Daniel et al, c/o S.A. LABAZ-SANOFI N.V. Avenue De Béjar, 1, B-1120 Bruxelles (BE)**

**EP 0 026 168 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés de décahydroquinoléinol, leur procédé de préparation ainsi que leurs applications en thérapeutique

La présente invention se rapporte, d'une manière générale, à de nouveaux composés hétérocycliques et, en particulier, à de nouveaux dérivés de décahydroquinoléinol ainsi qu'à leur procédé de préparation.

Les dérivés de décahydroquinoléinol de l'invention peuvent être représentés par la formule suivante:

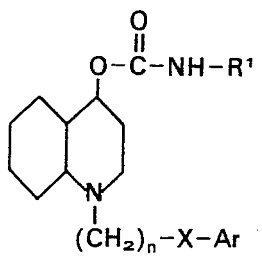

(I)

dans laquelle

$R^1$ représente hydrogène, un radical méthyle, éthyle, n-propyle, n-butyle, phényle ou un radical phényle mono- ou disubstitué par un atome de chlore, de brome ou par un radical méthyle,

Ar représente un radical phényle, naphtyle ou un radical phényle mono- ou disubstitué par un atome de fluor, de chore ou de brome ou par un groupement méthyle, méthoxy, acétyle ou cyano,

X représente oxygène ou soufre, et n représente 2 ou 3.

La présente invention se rapporte également aux sels d'addition non toxiques des dérivés de formule I, par exemple le chlorhydrate ou le méthanesulfonate.

Les composés de formule I possèdent, en position 4 du cycle décahydroquinoléine, un radical carbamoyloxy qui peut présenter une configuration axiale ou équatoriale. L'invention se rapporte à la fois aux épimères axiaux et équatoriaux en question et aux mélanges de ces épimères. On a découvert que les dérivés de décahydroquinoléinol de l'invention possèdent de remarquables propriétés pharmacologiques susceptibles de les rendre particulièrement utiles dans le traitement d'arythmies cardiaques de diverses origines et, en particulier, dans le traitement d'arythmies consécutives à l'infarctus du myocarde.

La dose efficace journalière sera, par exemple, de 100 à 200 mg par voie orale chez un être humain de 60 kg.

D'autre part, les composés de l'invention se sont révélés doués de très intéressantes propriétés anesthésiques locales.

Un autre objet de la présente invention se rapporte, en conséquence, à de nouveaux médicaments utiles notamment comme anesthésiques locaux et dans le traitement d'arythmies cardiaques de diverses origines, médicaments constitués par un dérivé de décahydroquinoléinol de formule I ou par un sel d'addition non toxique de ce dérivé.

Un autre objet de la présente invention concerne des compositions pharmaceutiques ou vétérinaires contenant, comme principe actif, au moins un dérivé de décahydroquinoléinol de formule I ou un sel d'addition non toxique de ce dérivé, en association avec un véhicule pharmaceutique ou un excipient approprié.

De même, l'invention se rapporte à un procédé de préparation de compositions pharmaceutiques ou vétérinaires, procédé suivant lequel on associe au moins un dérivé de décahydroquinoléinol de formule I ou un sel d'addition non toxique de ce dérivé à un véhicule pharmaceutique ou un excipient approprié.

Les composés de formule I peuvent être tous préparés au départ de l'hydroxy-4 transdécahydroquinoléine.

On fait tout d'abord réagir ce produit avec un composé halogéné de formule générale:

$$Hal-(CH_2)_n-X-Ar \qquad (II)$$

dans laquelle

Hal représente un atome de chlore, de brome ou d'iode, et

n, X et Ar ont la même signification que précédemment, ce qui fournit les hydroxy-4 transdécahydroquinoléines N-substituées de formule générale:

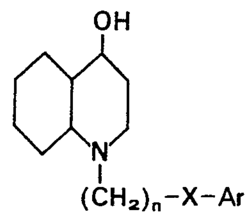

(III)

dans laquelle

n, X et Ar ont la même signification que précédemment. Cette réaction est effectuée, de préférence, soit dans un milieu alcoolique, par exemple le butanol, soit dans un milieu cétonique, par exemple la méthyléthylcétone, et en présence d'un accepteur d'acide, de préférence un carbonate de métal alcalin, par exemple le carbonate de potassium, le carbonate de sodium ou l'hydrogénocarbonate de sodium. La réaction, qui peut être accélérée au moyen de faibles quantités d'iodure de potassium, est conduite, de préférence, à la température de reflux du solvant.

On fait ensuite réagir dans un solvant organique inerte tel que, par exemple le benzène, le toluène ou le dichlorométhane et à la température ambiante, par exemple de 20 à 22°C, l'hydroxy-4 transdécahydroquinoléine N-substituée de formule III avec un composé de formule générale:

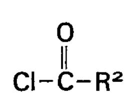

(IV)

dans laquelle

$R^2$ représente un atome de chlore ou un radical phénoxy pour obtenir un dérivé carbonyloxy de formule générale:

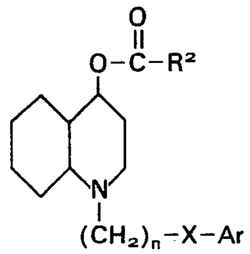

(V)

dans laquelle

n, X, $R^2$ et Ar ont la même signification que précédemment, ledit dérivé pouvant être condensé avec l'ammoniac ou une amine primaire de formule générale:

$$H_2N-R^1 \quad (VI)$$

dans laquelle

$R^1$ a la même signification que précédemment, de préférence dans un milieu inerte, par exemple le toluène ou le dichlorométhane ou un mélange de ces deux solvants et à la température ambiante pour former l'ester carbamique de décahydroquinoléinol correspondant de formule I sous la forme de sa base libre.

Les sels d'addition non toxiques des composés de formule I peuvent être préparés, de manière classique, en faisant réagir le composé de formule I correspondant sous la forme de sa base libre avec un acide organique tel que, par exemple, l'acide méthanesulfonique ou un acide inorganique tel que, par exemple, l'acide chlorhydrique.

Les composés halogénés de formule II peuvent être préparés par la méthode décrite par Ch. K. Grogan et coll. dans «J. Med. Chem.» 1965, *8*, 62, ou par d'autres procédés connus.

Quant à l'hydroxy-4 transdécahydroquinoléine de départ, il s'agit d'un produit connu pouvant être préparé en réduisant l'oxo-4 transdécahydroquinoléine suivant la méthode décrite, par exemple, dans «Bull. Acad. Sci.» USSR, *1962*, 1599.

Cette méthode conduit à un mélange d'épimères axial et équatorial au niveau de l'hydroxyle. Ces épimères, sous forme séparée, ont été décrits, ainsi que leurs préparation et identification, par M. Prost et coll. dans «Eur. J. Med. Chem.» 1976, *11* (4), pp. 337-342.

En conséquence, les procédés décrits ci-dessus pour la préparation des dérivés de formule I au départ d'hydroxy-4 transdécahydroquinoléine sont applicables soit à l'épimère axial, soit à l'épimère équatorial de l'hydroxy-4 transdécahydroquinoléine pour la préparation des épimères correspondants de formule I. De même, les procédés en question sont applicables au mélange d'épimères axial et équatorial d'hydroxy-4 transdécahydroquinoléine obtenu, par exemple, après réduction de l'oxo-4 transdécahydroquinoléine, en vue d'obtenir les composés de formule I sous la forme d'un mélange d'épimères axial et équatorial. Des composés de structure chimique semblable à celle des composés de formule I ci-dessus présentant des propriétés antiarythmiques sont déjà connus, ayant été publiés dans la demande de brevet français N° 2160939.

De tels dérivés se caractérisent par une chaîne (fluoro-4 phényl)-4 oxo-4 butyle en position 1 du cycle décahydroquinoléine.

S'il est exact que les dérivés de décahydroquinoléine en question sont doués de propriétés antiarythmiques, celles-ci, par contre, ne se manifestent que par voie veineuse. On a en effet remarqué que l'activité de ces produits par voie orale est très faible, ce qui les rend totalement sans valeur comme agents antiarythmiques lorsqu'ils sont administrés par voie orale. Par ailleurs, les dérivés antiarythmiques de la demande de brevet français en question sont susceptibles de provoquer, d'une part, d'importantes chutes de pression artérielle par suite de leur puissante activité antiadrénergique et, d'autre part, une réduction du débit cardiaque suite à la dépression qu'ils exercent sur la contractilité du myocarde.

Enfin, ils dépriment l'activité nerveuse centrale et sont, en particulier, sédatifs.

Tous ces inconvénients limitent donc l'utilisation de tels produits et nécessitent, en outre, un contrôle sévère du patient.

On a trouvé maintenant, de façon surprenante, qu'en modifiant légèrement la chaîne (fluoro-4 phényl)-4 oxo-4 butyle en question en y remplaçant la fonction carbonyle par une fonction étheroxyde, les nouveaux dérivés de décahydroquinoléinol ainsi obtenus présentent de remarquables propriétés anesthésiques locales provoquant, au niveau cardiaque, des changements électrophysiologiques et des effets antiarythmiques importants.

Ces propriétés antiarythmiques résultent, notamment, d'un mode d'action totalement différent de celui des dérivés du brevet français en question. D'autre part, des tests pharmacologiques ont démontré que les composés de l'invention sont beaucoup plus actifs, par voie orale, que les dérivés de décahydroquinoléinol du brevet français cité précédemment.

Par conséquent, les possibilités d'utilisation des dérivés de l'invention dans le domaine antiarythmique seront plus larges que celles offertes par les dérivés du brevet français N° 2160939; les composés de l'invention pourront être utilisés, par exemple, en traitement ambulatoire là où les dérivés de l'état de la technique feront évidemment défaut.

Parmi les composés de la présente invention qui ont montré les meilleures potentialités antiarythmiques, on peut citer tout particulièrement:

- la carbamoyloxy-4[(fluoro-4 phénoxy)-3 propyl]-1 transdécahydroquinoléine (forme axiale) ci-après dénommée composé A, et
- la [(chloro-3 méthyl-4 phényl)carbamoyloxy]-4 [(fluoro-4 phénoxy)-3 propyl]-1 transdécahydroquinoléine (forme équatoriale),

ces dérivés étant sous forme de base libre ou de sel d'addition non toxique tel que le chlorhydrate ou le méthanesulfonate.

Des tests pharmacologiques effectués plus particulièrement avec le composé A ont montré qu'il possède l'ensemble des propriétés souhaitables en vue de son utilisation dans le traitement d'arythmies d'origines diverses.

Ainsi, on a pu mettre en évidence, chez le composé A, une activité antiarythmique très polyvalente puisqu'elle se manifeste à la fois aux niveaux auriculaire et ventriculaire suivant les différents types d'arythmies cardiaquès provoquées expérimentalement. Par exemple, le composé A est actif dès la dose de 1 à 2,5 mg/kg par voie intraveineuse chez le chien vis-à-vis des arythmies ventriculaires induites par l'épinéphrine, le chlorure de baryum ou l'ouabaïne. Il protège également contre la fibrillation ventriculaire provoquée par le chloroforme ou par le chlorure de calcium et contre la fibrillation auriculaire provoquée par l'acétylcholine.

D'autre part, le composé A n'est ni hypotenseur, son mécanisme d'action ne résultant pas de propriétés antiadrénergiques, ni dépresseur de la contractilité du myocarde. Il n'est pas non plus arythmogène aux doses toxiques puisque, pendant une perfusion veineuse continue chez le chien anesthésié, la dose arythmisante est de $80,6 \pm 6,9$ mg/kg et la dose mortelle de $84,1 \pm 7,1$ mg/kg.

Enfin, il n'affecte pas la vigilance, puisqu'il ne modifie pas l'activité de la souris.

A la lumière de ces différentes propriétés, on peut considérer que les composés de l'invention et, en particulier le composé A, constituent une source précieuse de substances potentiellement efficaces pour le traitement d'arythmies d'étiologies diverses, par exemple pour le traitement de cas de fibrillation auriculaire, en particulier d'origine parasympathique.

En outre, les composés de l'invention pourront être utilisés efficacement pour le traitement, par exemple d'arythmies post-infarctus, ce qui leur conférerait, par conséquent, un avantage incontestable pour prévenir la mort subite par fibrillation ventriculaire après infarctus du myocarde.

Parmi les agents les plus efficaces utilisés dans le traitement des arythmies post-infarctus, la lidocaïne est probablement l'un des plus courants.

Cependant, ce composé n'est utile que par voie veineuse comme agent antiarythmique, sa demi-vie plasmatique chez l'être humain étant très courte, de l'ordre de 15 min.

S'il est difficile, par conséquent, de dépasser des doses toxiques, il est, par contre, malaisé de régler la vitesse de perfusion de ce produit pour en stabiliser le taux plasmatique.

Les composés de l'invention ne présentent pas ce désavantage car, au contraire de la lidocaïne, ceux-ci sont actifs par voie orale, comme mentionné précédemment, et d'une durée d'action plus prolongée. Ce double avantage représente un progrès pharmacologique important par rapport à la lidocaïne: le taux plasmatique d'agent antiarythmique selon l'invention sera plus aisé à stabiliser dans le temps, et les traitements ambulatoires deviendront possibles, ne nécessitant plus l'hospitalisation du patient.

On trouvera, par la suite, des résultats obtenus au cours de l'étude pharmacologique des composés de l'invention

A. *Propriétés antiarythmiques*

Celles-ci ont été mises en évidence au moyen du test de Lawson («J. Pharmac. Exp. Therap.» 1968, *160* (1), 22-31).

L'arythmie est provoquée, dans ce test, par l'inhalation de chloroforme jusqu'à l'arrêt respiratoire.

Pour chaque dose de substance à étudier, on utilise des lots de 10 souris femelles de 20 g environ, à jeun de nourriture depuis 18 h avant l'essai, un lot de 10 animaux servant de lot témoin.

On administre d'abord, par voie intragastrique, une dose du composé à étudier, excepté chez les témoins qui ne reçoivent que le solvant du composé en question.

30 min plus tard, on place les animaux dans une cloche saturée de chloroforme grâce à un tampon fortement imbibé. On retire l'animal dès asphyxie totale (environ 2 min plus tard), on ouvre rapidement le thorax et on observe le rythme ventriculaire.

On détermine alors la dose de substance à étudier qui protège 50% des animaux contre la fibrillation ventriculaire.

Cette dose protectrice est exprimée comme étant la $DA_{50}$ en mg/kg dans le tableau suivant. Les composés de formule I ont été utilisés sous la forme d'un sel d'addition non toxique, essentiellement sous forme de chlorhydrate en comparaison avec la lidocaïne.

*Tableau*

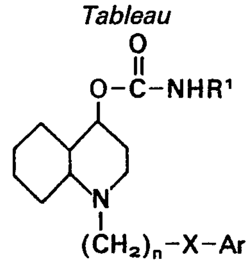

| $R^1$ | n | X | Ar | Stéréochimie du OCO-NHR[1] | $DA_{50}$ (mg/kg) |
|---|---|---|---|---|---|
| hydrogène | 3 | 0 | fluoro-4 phényle | axiale | 45 |
| méthyle | 3 | 0 | fluoro-4 phényle | axiale | 55 |
| éthyle | 3 | 0 | fluoro-4 phényle | axiale | 50 |

| $R^1$ | n | X | Ar | Stéréochimie du OCO-NHR$^1$ | $DA_{50}$ (mg/kg) |
|---|---|---|---|---|---|
| n-propyle | 3 | 0 | fluoro-4 phényle | axiale | 80 |
| n-butyle | 3 | 0 | fluoro-4 phényle | axiale | 50 |
| phényle | 3 | 0 | fluoro-4 phényle | axiale | >100 |
| bromo-4 phényle | 3 | 0 | fluoro-4 phényle | axiale | 150 |
| chloro-3 méthyl-4 phényle | 3 | 0 | fluoro-4 phényle | axiale | >100 |
| hydrogène | 3 | 0 | phényle | axiale | >100 |
| hydrogène | 3 | 0 | bromo-4 phényle | axiale | 65 |
| hydrogène | 3 | 0 | chloro-4 phényle | axiale | >100 |
| hydrogène | 3 | 0 | méthyl-4 phényle | axiale | >100 |
| hydrogène | 3 | 0 | méthoxy-4 phényle | axiale | >100 |
| hydrogène | 3 | 0 | acétyl-4 phényle | axiale | >100 |
| hydrogène | 3 | 0 | cyano-4 phényle | axiale | 30 |
| hydrogène | 3 | 0 | méthoxy-2 acétyl-4 phényle | axiale | >100 |
| bromo-4 phényle | 3 | 0 | méthoxy-2 acétyl-4 phényle | axiale | >100 |
| hydrogène | 3 | 0 | naphtyl-1 | axiale | >100 |
| hydrogène | 3 | 0 | naphtyl-2 | axiale | >100 |
| hydrogène | 2 | 0 | fluoro-4 phényle | axiale | >100 |
| hydrogène | 2 | 0 | bromo-4 phényle | axiale | 90 |
| hydrogène | 2 | 0 | dichloro-2,6 phényle | axiale | 50 |
| hydrogène | 2 | 0 | méthoxy-2 phényle | axiale | 75 |
| hydrogène | 2 | 0 | naphtyl-1 | axiale | 75 |
| hydrogène | 2 | 0 | naphtyl-2 | axiale | 50 |
| hydrogène | 3 | S | fluoro-4 phényle | axiale | >100 |
| hydrogène | 3 | 0 | fluoro-4 phényle | équatoriale | 45 |
| bromo-4 phényle | 3 | 0 | fluoro-4 phényle | équatoriale | 120 |
| chloro-3 méthyl-4 phényle | 3 | 0 | fluoro-4 phényle | équatoriale | 30 |
| lidocaïne | | | | | 35 |

Un autre test comparatif a été effectué dans les mêmes conditions avec quelques composés couverts par la demande de brevet français No 2160939 à la dose unique de 100 mg/kg par voie intragastrique.

On a obtenu les résultats suivants (exprimés en pourcentage de protection contre l'effet arythmique à la dose considérée):

| $R^1$ | n | X | Ar | Stéréochimie du OCO-NHR$^1$ | Protection (%) |
|---|---|---|---|---|---|
| hydrogène | 3 | $\overset{O}{\overset{\parallel}{C}}$ | fluoro-4 phényle | axiale | 35 |
| méthyle | 3 | $\overset{O}{\overset{\parallel}{C}}$ | fluoro-4 phényle | axiale | 35 |
| éthyle | 3 | $\overset{O}{\overset{\parallel}{C}}$ | fluoro-4 phényle | axiale | 30 |
| n-propyle | 3 | $\overset{O}{\overset{\parallel}{C}}$ | fluoro-4 phényle | axiale | 0 |
| bromo-4 phényle | 3 | $\overset{O}{\overset{\parallel}{C}}$ | fluoro-4 phényle | axiale | 0 |
| hydrogène | 3 | $\overset{O}{\overset{\parallel}{C}}$ | fluoro-4 phényle | équatoriale | 35 |

Ces résultats montrent parfaitement que le remplacement de la fonction carbonyle par une fonction éther-oxyde dans la chaîne située en position 1 du cycle décahydroquinoléine augmente considérablement l'activité antiaryhtmique par voie intragastrique.

D'autre part, si, dans ce test, la plupart des composés de l'invention sont moins actifs que la

lidocaïne 30 min après leur administration, leur durée d'action, par contre, est beaucoup plus prolongée. Ainsi, 75 mg/kg soit de composé A, soit de lidocaïne, par voie intragastrique chez la souris, ont fourni les résultats suivants:

| Temps après administration (min) | Protection (%) | |
|---|---|---|
| | Composé A | Lidocaïne |
| 30 | 80 | 95 |
| 60 | 65 | 65 |
| 120 | 60 | 25 |
| 180 | 45 | — |
| 240 | 30 | — |

B. *Propriétés antiadrénergiques et sédatives*

Des tests effectués avec le composé A ont montré qu'une dose de 50 mg/kg administrée par voie intragastrique chez la souris 30 min avant l'injection intraveineuse de 3 mg/kg d'épinéphrine n'offre aucune protection contre la toxicité de cette catécholamine. De même, une dose intragastrique de 50 mg/kg de composé A administrée à des souris n'engendre pas de dépression de la motilité spontanée 30 min plus tard. Ces résultats démontrent que le composé A n'est ni sédatif ni antiadrénergique aux doses antiarythmiques.

C. *Propriétés anesthésiques locales*

Celles-ci ont été mises en évidence sur cobaye par la technique de Chance et Lobstein, laquelle consiste à déposer une goutte d'une solution du composé à étudier dans l'œil de l'animal et à vérifier, différents temps après administration, s'il y a fermeture ou pas de la paupière après contact de la cornée. On détermine ainsi le pourcentage d'animaux anesthésiés localement en fonction de la dose de composé et en fonction du temps après administration.

On a remarqué qu'une solution du composé A à la concentration de 0,1% anesthésie localement 58% des animaux 5 min après administration, alors qu'une même concentration en lidocaïne, 5 min après administration, n'anesthésie localement que 26% des cobayes.

D'autre part, une solution de lidocaïne à la concentration de 0,5% ne protège plus que 35% des animaux 15 min après administration, alors qu'une même concentration de composé A protège encore 35% des cobayes 30 min après administration.

Ces résultats montrent que le composé A est plus actif que la lidocaïne comme anesthésique local et de durée d'action plus importante.

D. *Toxicité*

Des tests de toxicité aiguë ont été pratiqués avec le composé A chez la souris et le rat.

Par voies intraveineuse et intragastrique, la $DL_{50}$ du composé A, c'est-à-dire la dose nécessaire pour tuer 50% des animaux, est respectivement de 42,5 mg/kg et 750 mg/kg chez la souris. Chez le rat, par voie intraveineuse et intragastrique, la $DL_{50}$ est respectivement de 47 mg/kg et de plus de 2000 mg/kg.

Par rapport au composé A, la lidocaïne s'est révélée plus toxique, la $DL_{50}$ par voies intraveineuse et intragastrique chez le rat étant respectivement de 12,5 et 360 mg/kg.

De plus, le composé A possède une bonne marge de sécurité entre sa dose efficace antiarythmique et sa dose convulsivante, comme en témoigne le tableau suivant:

| | Dose (mg/kg) | |
|---|---|---|
| | Antiarythmique | Convulsivante |
| Souris (p.o.) | 45 ($AD_{50}$ Test Lawson) | 300 à 400 ($AD_0$) |
| Chien (i.v.) | 1 à 2,5 (chien anesthésié) | 6 à 10 (chien conscient) |

Les compositions thérapeutiques selon l'invention peuvent être présentées sous toute forme convenant à l'administration en thérapie humaine ou vétérinaire. Pour ce qui concerne l'unité d'administration, celle-ci peut prendre la forme, par exemple, d'un comprimé, d'une dragée, d'une capsule, d'une gélule, d'une poudre, d'une suspension ou d'un sirop pour l'administration orale, d'un suppositoire pour l'administration rectale, d'une solution ou suspension pour l'administration parentérale ou d'une crème, d'un onguent, d'une lotion ou d'un gel pour l'administration topique.

Suivant la voie d'administration choisie, les compositions thérapeutiques de l'invention seront préparées en associant au moins un composé de formule I ou un sel d'addition non toxique de ce composé avec un excipient approprié, ce dernier pouvant être constitué, par exemple, d'au moins un ingrédient sélectionné parmi les substances suivantes: eau distillée, alcool benzylique, lactose, amidons, talc, stéarate de magnésium, polyvinylpyrrolidone, phosphate monopotassique, phosphate disodique dihydraté, acide alginique, silice colloïdale, polyéthylèneglycol ou agents édulcorants.

La préparation des composés de l'invention, ainsi que la formulation des compositions thérapeutiques dont il est question ci-dessus, sont illustrées par les exemples non limitatifs suivants:

*Exemple 1:*

*Carbamoyloxy-4 [(fluoro-4 phénoxy)-3 propyl]-1 transdécahydroquinoléine (forme axiale) et son chlorhydrate*

a) *[(Fluoro-4 phénoxy)-3 propyl]-1 hydroxy-4 transdécahydroquinoléine (forme axiale) et son chlorhydrate*

On chauffe à reflux pendant 48 h, une solution de 12,5 g (0,08 mol) d'hydroxy-4 transdécahydroquinoléine (forme axiale) et de 16,6 g

(0,088 mol) de (chloro-3 propoxy)-1 fluoro-4 benzène dans 120 ml de butanol-1 en présence de 9 g d'hydrogénocarbonate de sodium. L'eau formée durant la réaction est éliminée par distillation azéotropique. Après refroidissement, on filtre les sels, on lave le filtre avec un peu de butanol-1 et on évapore le filtrat à sec. On reprend le résidu avec 150 ml d'éther diéthylique et on acidifie la solution ainsi formée avec de l'acide chlorhydrique à 10%.

On décante la phase aqueuse et on l'alcalinise avec de l'hydroxyde de sodium à 10%. On réextrait avec de l'éther diéthylique et on lave la phase éthérée à l'eau. Après séchage sur du sulfate de sodium et filtration on élimine le solvant. Le résidu d'évaporisation cristallise en présence de 75 ml de n-hexane. On filtre les cristaux et on lave le filtre avec un peu de n-hexane. On isole ainsi 18 g de [(fluoro-4 phénoxy)-3 propyl]-1 hydroxy-4 transdécahydroquinoléine (forme axiale) sous forme de base libre.

Rendement: 65%

PF: 95±1 °C

On dissout ensuite la base ainsi obtenue dans du propanol-2 et on ajoute une quantité stœchiométrique d'acide chlorhydrique gazeux dissous dans du propanol-2. Après élimination du solvant, on recristallise dans un mélange d'acétate d'éthyle et de méthanol.

De cette manière, on obtient le chlorhydrate de [(fluoro-4 phénoxy)-3 propyl]-1 hydroxy-4 transdécahydroquinoléine (forme axiale). PF: 167-170° C.

Au départ des produits appropriés et en utilisant le procédé décrit ci-dessus, on a préparé les composés suivants: pour chaque composé de forme axiale, le solvant de recristallisation a été un mélange d'acétate d'éthyle et de méthanol.

| Composé | PF (°C) |
|---|---|
| Chlorhydrate d'hydroxy-4 (phénoxy-3 propyl)-1 transdécahydroquinoléine (forme axiale) | 170±1 |
| Chlorhydrate de [(bromo-4 phénoxy)-3 propyl]-1 hydroxy-4 transdécahydroquinoléine (forme axiale) | 182±1 |
| Chlorhydrate de [(chloro-4 phénoxy)-3 propyl]-1 hydroxy-4 transdécahydroquinoléine (forme axiale) | 167±1 |
| Chlorhydrate d'hydroxy-4 [(méthoxy-4 phénoxy)-3 propyl]-1 transdécahydroquinoléine (forme axiale) | 136±1 |
| Chlorhydrate de [(cyano-4 phénoxy)-3 propyl]-1 hydroxy-4 transdécahydroquinoléine (forme axiale) | 162±11 |
| Chlorhydrate d'hydroxy-4 [(naphtyl-1 oxy)-3 propyl]-1 transdécahydroquinoléine (forme axiale) | 154±1 |
| Chlorhydrate d'hydroxy-4 [(naphtyl-2 oxy)-3 propyl]-1 transdécahydroquinoléine (forme axiale) | 187±1 |
| Chlorhydrate d'[(acétyl-4 méthoxy-2 phénoxy)-3 propyl]-1 hydroxy-4 transdécahydroquinoléine (forme axiale) | 170±1 |
| Chlorhydrate de [(fluoro-4 phénylthio)-3 propyl]-1 hydroxy-4 transdécahydroquinoléine (forme axiale) | 157±1 |
| Chlorhydrate de [(cyano-2 phénoxy)-2 éthyl]-1 hydroxy-4 transdécahydroquinoléine (forme axiale) | 212±1 |
| Chlorhydrate d'hydroxy-4 [(naphtyl-1 oxy)-2 éthyl]-1 transdécahydroquinoléine (forme axiale) | 246±1 |
| Chlorhydrate d'hydroxy-4 [(naphtyl-2 oxy)-2 éthyl]-1 transdécahydroquinoléine (forme axiale) | 188±1 |
| Chlorhydrate de [(fluoro-4 phénoxy)-3 propyl]-1 hydroxy-4 transdécahydroquinoléine (forme équatoriale) | 99±1 (cyclohexane) |

b) *Carbamoyloxy-4 [(fluoro-4 phénoxy)-3 propyl]-1 transdécahydroquinoléine (forme axiale) et son chlorhydrate*

A la température de 0 à 5° C, on prépare une solution de 200 g de phosgène dans 1 l de toluène anhydre. On y introduit ensuite, entre 0 et −10° C, une solution de 278 g (0,9 mol) de [(fluoro-4 phénoxy)-3 propyl]-1 hydroxy-4 transdécahydroquinoléine (forme axiale) dans 1,5 l de dichlorométhane.

L'addition terminée, on maintient le mélange réactionnel sous agitation à une température de 20 à 22° C pendant 2 d. On élimine l'excès de phosgène en soumettant le mélange réactionnel au vide d'une trompe à eau, pendant 2 h, tout en maintenant une température de 20 à 25° C par un léger chauffage.

On note un début de cristallisation et on réajuste le volume avec du dichlorométhane.

On refroidit le milieu réactionnel vers 5° C et on le sature avec de l'ammoniac. On maintient le mélange pendant 48 h sous agitation, ensuite on introduit une quantité supplémentaire de 1,2 l d'ammoniaque concentrée.

On agite à nouveau durant 24 h à température ambiante (21±2° C) et on décante la phase

aqueuse. On évapore le solvant de la phase organique et on réempâte, dans 1 l d'hexane, le résidu solidifié. On filtre ensuite les cristaux de carbamoyloxy-4 [(fluoro-4 phénoxy)-3 propyl]-1 transdécahydroquinoléine (forme axiale) sous forme de base libre, ainsi obtenus.

PF: 116±1 °C.

Après séchage, on dissout la base ainsi obtenue dans 2 l d'acétate d'éthyle et on prépare le chlorhydrate par addition d'une quantité stœchiométrique d'acide chlorhydrique gazeux dissous dans du propanol-2.

Le chlorhydrate désiré précipite. On le filtre et on lave le filtre à l'acétone. Après séchage, on recristallise ce produit dans un mélange 6/4 d'acétate d'éthyle/méthanol, puis on le sèche.

De cette manière, on obtient 278,5 g de chlorhydrate de carbamoyloxy-4 [(fluoro-4 phénoxy)-3 propyl]-1 transdécahydroquinoléine (forme axiale).

Rendement: 80%

PF: 238±1 °C

Au départ des produits appropriés et en utilisant le procédé décrit ci-dessus, on a préparé les composés suivants:

| Composé | PF (°C) |
|---|---|
| Chlorhydrate de carbamoyloxy-4 (phénoxy-3 propyl)-1 transdécahydroquinoléine (forme axiale) | 252±2 (acétate d'éthyle/méthanol) |
| Chlorhydrate de [(bromo-4 phénoxy)-3 propyl]-1 carbamoyloxy-4 transdécahydroquinoléine (forme axiale) | 257±2 (méthanol) |
| Chlorhydrate de carbamoyloxy-4 [(chloro-4 phénoxy)-3 propyl]-1 transdécahydroquinoléine (forme axiale) | 236±1 (méthanol) |
| Chlorhydrate de carbamoyloxy-4 [(méthyl-4 phénoxy)-3 propyl]-1 transdécahydroquinoléine (forme axiale) | 244±2 (méthanol) |
| Chlorhydrate de carbamoyloxy-4 [(méthoxy-4 phénoxy)-3 propyl]-1 transdécahydroquinoléine (forme axiale) | 240±2 (méthanol) |
| Chlorhydrate d'[(acétyl-4 phénoxy)-3 propyl]-1 carbamoyloxy-4 transdécahydroquinoléine (forme axiale) | 254±2 (acétate d'éthyle/méthanol) |
| Chlorhydrate de carbamoyloxy-4 [(cyano-4 phénoxy-3 propyl]-1 transdécahydroquinoléine (forme axiale) | 162±1 (méthanol) |
| Chlorhydrate de carbamoyloxy-4 [(naphtyl-1 oxy)-3 propyl]-1 transdécahydroquinoléine (forme axiale) | 259±1 (acétone) |
| Chlorhydrate de carbamoyloxy-4 [(naphtyl-2 oxy)-3 propyl]-1 transdécahydroquinoléine (forme axiale) | >260 (acétone) |
| Chlorhydrate de carbamoyloxy-4 [(fluoro-4 phénylthio)-3 propyl]-1 transécahydroquinoléine (forme axiale) | 215±1 (acétate d'éthyle/méthanol) |
| Chlorhydrate de [(fluoro-4 phénoxy)-3 propyl]-1 (N-méthylcarbamoyloxy)-4 transdécahydroquinoléine (forme axiale) | 220±1 (méthanol) |
| Chlorhydrate de (N-éthylcarbamoyloxy)-4 [(fluoro-4 phénoxy)-3 propyl]-1 transdécahydroquinoléine (forme axiale) | 174±1 (acétone) |
| Chlorhydrate de [(fluoro-4 phénoxy)-3 propyl]-1 (N-n-propylcarbamoyloxy)-4 transdécahydroquinoléine (forme axiale) | 159±1 (acétone) |
| Chlorhydrate de (N-n-butylcarbamoyloxy)-4 [(fluoro-4 phénoxy)-3 propyl]-1 transdécahydroquinoléine (forme axiale) | 140±1 (acétone) |
| Chlorhydrate de [(fluoro-4 phénoxy)-3 propyl]-1 (N-phénylcarbamoyloxy)-4 transdécahydroquinoléine (forme axiale) | 219±1 (acétate d'éthyle/méthanol) |
| Chlorhydrate de [(bromo-4 phényl) carbamoyloxy]-4 [(fluoro-4 phénoxy)-3 propyl]-1 transdécahydroquinoléine (forme axiale) | 173±1 (acétate d'éthyle) |
| Chlorhydrate de [(chloro-3 méthyl-4 phényl)carbamoyloxy]-4 [(fluoro-4 phénoxy)-3 propyl]-1 transdécahydroquinoléine (forme axiale) | 176±1 (acétate d'éthyle) |
| Chlorhydrate d'[(acétyl-4 méthoxy-2 phénoxy)-3 propyl]-1 carbamoyloxy-4 transdécahydroquinoléine (forme axiale) | 239±1 (acétate d'éthyle/méthanol) |
| Chlorhydrate d'[(acétyl-4 méthoxy-2 phénoxy)-3 propyl]-1 [(bromo-4 phényl) carbamoyloxy]-4 transdécahydroquinoléine (forme axiale) | 160±1 (acétate d'éthyle/méthanol) |
| Chlorhydrate de carbamoyloxy-4 [(dichloro-2,6 phénoxy)-2 éthyl]-1 transdécahydroquinoléine (forme axiale) | >260 (acétone) |
| Chlorhydrate de carbamoyloxy-4 [(cyano-2 phénoxy)-2 éthyl]-1 transdécahydroquinoléine (forme axiale) | 206±1 (acétone) |
| Chlorhydrate de carbamoyloxy-4 [(méthoxy-2 phénoxy)-2 éthyl]-1 transdécahydroquinoléine (forme axiale) | 226±1 (acétate d'éthyle/méthanol) |
| Chlorhydrate de carbamoyloxy-4 [(fluoro-4 phénoxy)-2 éthyl]-1 transdécahydroquinoléine (forme axiale) | 223±1 (acétate d'éthyle/méthanol) |
| Chlorhydrate de [(bromo-4 phénoxy)-2 éthyl]-1 carbamoyloxy-4 transdécahydroquinoléine (forme axiale) | ±250 (méthanol) |

| Composé | PF (°C) |
|---|---|
| Chlorhydrate de carbamoyloxy-4 [(naphtyl-1 oxy)-2 éthyl]-1 transdécahydroquinoléine (forme axiale) | 185 (méthanol/eau/acétate d'éthyle) |
| Chlorhydrate de carbamoyloxy-4 [(naphtyl-2 oxy)-2 éthyl]-1 transdécahydroquinoléine (forme axiale) | >260 (méthanol/eau/acétate d'éthyle) |
| Chlorhydrate de carbamoyloxy-4 [(fluoro-4 phénoxy)-3 propyl]-1 transdécahydroquinoléine (forme équatoriale) | 211±1 (acétate d'éthyle/méthanol) |
| Chlorhydrate de [(bromo-4 phényl) carbamoyloxy]-4 [(fluoro-4 phénoxy)-3 propyl]-1 transdécahydroquinoléine (forme équatoriale) | 241±1 (acétate d'éthyle/méthanol) |
| Chlorhydrate de [(chloro-3 méthyl-4 phényl)carbamoyloxy]-4 [(fluoro-4 phénoxy)-3 propyl]-1 transdécahydroquinoléine (forme équatoriale) | 229±1 (acétate d'éthyle/méthanol) |

*Exemple 2:*

*Méthanesulfonate de carbamoyloxy-4 [(fluoro-4 phénoxy)-3 propyl]-1 transdécahydroquinoléine (forme axiale)*

On ajoute à une solution de 4 g (0,0114 mol) de carbamoyloxy-4 [(fluoro-4 phénoxy)-3 propyl]-1 transdécahydroquinoléine (forme axiale) préparée comme décrit dans l'exemple 1, dans 30 ml d'acétone, la quantité stœchiométrique d'acide méthanesulfonique en solution aqueuse à 70% dissous dans 30 ml de propanol-2.

On évapore à sec le mélange réactionnel et on élimine l'eau par plusieurs distillations azéotropiques avec du toluène. On dissout ensuite le résidu huileux dans de l'acétate d'éthyle et on laisse cristalliser. On filtre et on lave le filtre à l'acétate d'éthyle.

De cette manière, on isole 3 g de méthanesulfonate de carbamoyloxy-4 [(fluoro-4 phénoxy)-3 propyl]-1 transdécahydroquinoléine (forme axiale).
PF: 238±1° C.

*Exemple 3:*

*Chlorhydrate de carbamoyloxy-4 [(fluoro-4 phénoxy)-3 propyl]-1 transdécahydroquinoléine (forme axiale)*

a) *[(Fluoro-4 phénoxy)-3 propyl]-1 hydroxy-4 transdécahydroquinoléine (forme axiale)*

On chauffe à reflux pendant 48 h avec élimination azéotropique de l'eau formée, 12,5 g (0,08 mol) d'hydroxy-4 transdécahydroquinoléine (forme axiale), 19 g (0,082 mol) de (bromo-3 propoxy)-1 fluoro-4 benzène et 9 g d'hydrogénocarbonate de sodium dans 100 ml de butanol-1.

Les conditions opératoires ultérieures sont identiques à celles décrites dans l'exemple 1.

De cette manière, on isole 18,5 g de [(fluoro-4 phénoxy)-3 propyl]-1 hydroxy-4 transdécahydroquinoléine (forme axiale) sous forme de base libre.
Rendement: 76%
PF: 95±1 °C

b) *Chlorhydrate de carbamoyloxy-4 [(fluoro-4 phénoxy)-3 propyl]-1 transdécahydroquinoléine (forme axiale)*

Sous agitation, on ajoute, goutte à goutte, 1,2 g (0,0075 mol) de phényle chloroformiate à une solution de 1,5 g (0,005 mol) de [(fluoro-4 phénoxy)-3 propyl]-1 hydroxy-4 transdécahydroquinoléine (forme axiale) dans 0,9 ml de pyridine et 15 ml de benzène, en prenant soin de maintenir la température entre 0 et 20° C durant l'opération. On poursuit l'agitation pendant 48 h à température ambiante, puis on verse le mélange réactionnel dans une solution aqueuse de carbonate de sodium. On extrait avec du benzène et on décante la phase organique. On évapore le solvant et on reprend le résidu par 10 ml de méthanol saturé en ammoniac.

Après 48 h à température ambiante, on évapore le solvant, on dissout le résidu dans du benzène et on lave cette solution à l'eau, ensuite avec une solution diluée d'hydroxide de sodium et finalement à l'eau. On évapore le solvant et on reprend le résidu dans l'acétate d'éthyle. Les conditions opératoires ultérieures sont identiques à celles décrites dans l'exemple 1.

De cette manière, on obtient 1,2 g de chlorhydrate de carbamoyloxy-4 [(fluoro-4 phénoxy)-3 propyl]-1 transdécahydroquinoléine (forme axiale).

PF: 238±1 °C

*Exemple 4:*

Suivant des techniques pharmaceutiques connues, on a préparé une gélule par association des ingrédients suivants:

| Ingrédients | mg |
|---|---|
| Chlorhydrate de carbamoyloxy-4 [(fluoro-4 phénoxy)-3 propyl]-1 transdécahydroquinoléine (forme axiale) | 100 |
| Amidon de maïs | 384 |
| Talc | 10 |
| Silice colloïdale | 6 |
| | 500 |

*Exemple 5:*

Suivant des techniques pharmaceutiquement connues, on a préparé un comprimé et des dragées par granulation et compression des ingrédients suivants:

a) *Comprimé*

| Ingrédients | mg |
|---|---|
| Chlorhydrate de carbamoyloxy-4 [(fluoro-4 phénoxy)-3 propyl]-1 trans-décahydroquinoléine (forme axiale) | 100 |
| Lactose | 128 |
| Polyvinylpyrrolidone | 12 |
| Carboxyméthylamidon sodique | 48 |
| Stéarate de magnésium | 8 |
| Talc | 4 |
| | 300 |

b) *Dragée*

On a préparé une dragée au départ du noyau de l'exemple ci-dessus en y ajoutant le vernis suivant:

| | |
|---|---|
| Polyacrylate cationique | ± 9 mg |
| Polyéthylèneglycol 6000 | ± 4 mg |
| Talc | ±40 mg |
| Hydroxypropylcellulose | ± 0,4 mg |
| Oxyde de titane | ±26,6 mg |
| | ±80 mg |

c) *Dragée entérosoluble*

On a préparé une dragée entérique au départ de la dragée de l'exemple ci-dessus en y ajoutant l'enrobage entérique suivant:

| | |
|---|---|
| Acétophtalate de cellulose | 40 mg |
| Diéthylphtalate | 10 mg |

*Exemple 6:*

Suivant des techniques pharmaceutiques connues, on a préparé une solution injectable à partir des ingrédients suivants:

| Ingrédients | |
|---|---|
| Chlorhydrate de carbamoyloxy-4 [(fluoro-4 phénoxy)-3 propyl]-1 transdécahydroquinoléine (forme axiale) | 200 mg |
| Mannitol | 800 mg |
| Eau distillée q.s.p. | 20 ml |

**Revendications**

1. Dérivés de décahydroquinoléinol correspondant à la formule générale

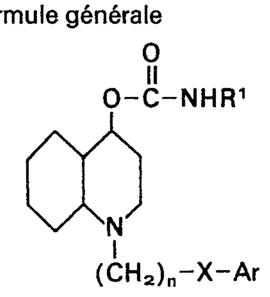

ainsi que leurs sels d'addition non toxiques, dans laquelle

$R^1$ représente l'hydrogène, un radical méthyle, éthyle, n-propyle, n-butyle, phényle ou un radical phényle mono- ou disubstitué par un atome de chlore, de brome ou par un radical méthyle,

Ar représente un radical phényle, naphtyle ou un radical phényle mono- ou disubstitué par un atome de fluor, de chlore ou de brome ou par un groupement méthyle, méthoxy, acétyle ou cyano,

X représente oxygène ou soufre, et

n représente 2 ou 3.

2. Dérivés de décahydroquinoléinol selon la revendication 1 sous forme d'épimères axiaux.

3. Dérivés de décahydroquinoléinol selon la revendication 1 sous forme d'épimères équatoriaux.

4. Carbamoyloxy-4 [(fluoro-4 phénoxy)-3 propyl]-1 transdécahydroquinoléine (forme axiale) et ses sels d'addition non toxiques.

5. [(Bromo-4 phénoxy)-3 propyl]-1 carbamoyloxy-4 transdécahydroquinoléine (forme axiale) et ses sels d'addition non toxiques.

6. Carbamoyloxy-4 [(cyano-4 phénoxy)-3 propyl]-1 transdécahydroquinoléine (forme axiale) et ses sels d'addition non toxiques.

7. [(Fluoro-4 phénoxy)-3 propyl]-1 (N-méthylcarbamoyloxy)-4 transdécahydroquinoléine (forme axiale) et ses sels d'addition non toxiques.

8. (N-Ethylcarbamoyloxy)-4 [(fluoro-4 phénoxy)-3 propyl]-1 transdécahydroquinoléine (forme axiale) et ses sels d'addition non toxiques.

9. [(Fluoro-4 phénoxy)-3 propyl]-1 (N-n-propylcarbamoyloxy)-4 transdécahydroquinoléine (forme axiale) et ses sels d'addition non toxiques.

10. (N-n-Butylcarbamoyloxy)-4 [(fluoro-4 phénoxy)-3 propyl]-1 transdécahydroquinoléine (forme axiale) et ses sels d'addition non toxiques.

11. Carbamoyloxy-4 [(dichloro-2,6 phénoxy)-2 éthyl]-1 transdécahydroquinoléine (forme axiale) et ses sels d'addition non toxiques.

12. Carbamoyloxy-4 [(fluoro-4 phénoxy)-3 propyl]-1 transdécahydroquinoléine (forme équatoriale) et ses sels d'addition non toxiques.

13. [(Chloro-3 méthyl-4 phényl)carbamoyloxy]-4 [(fluoro-4 phénoxy)-3 propyl]-1 transdécahydroquinoléine (forme équatoriale) et ses sels d'addition non toxiques.

14. [(Bromo-4 phénoxy)-2 éthyl]-1 carbamoyloxy-4 transdécahydroquinoléine (forme axiale) et ses sels d'addition non toxiques.

15. Carbamoyloxy-4 [(naphtyl-1 oxy)-2 éthyl]-1 transdécahydroquinoléine (forme axiale) et ses sels d'addition non toxiques.

16. Carbamoyloxy-4 [(naphtyl-2 oxy)-2 éthyl]-1 transdécahydroquinoléine (forme axiale) et ses sels d'addition non toxiques.

17. Dérivés de décahydroquinoléinol selon l'une quelconque des revendications 1 à 16, caractérisés en ce que le sel d'addition non toxique est le chlorhydrate ou le méthanesulfonate.

18. Nouveau médicament, caractérisé en ce que le principe actif est constitué par un dérivé de

décahydroquinoléinol selon l'une quelconque des revendications 1 à 16.

19. Nouveau médicament utile dans les arythmies cardiaques d'origines diverses, en particulier dans les arythmies consécutives à l'infarctus du myocarde, caractérisé en ce qu'il est constitué par un dérivé de décahydroquinoléinol selon l'une quelconque des revendications 1 à 16.

20. Nouveau médicament utile pour provoquer une anesthésie locale, caractérisé en ce qu'il est constitué par un dérivé de décahydroquinoléinol selon l'une quelconque des revendications 1 à 16.

21. Composition pharmaceutique ou vétérinaire, caractérisée en ce qu'elle contient, à titre de principe actif, au moins un dérivé de décahydroquinoléinol selon l'une quelconque des revendications 1 à 3, en association avec un véhicule pharmaceutique ou un excipient approprié.

22. Procédé de préparation de dérivés de décahydroquinoléinol selon la revendication 1, caractérisé en ce que l'on fait réagir, dans un solvant organique inerte et à la température ambiante, une hydroxy-4 transdécahydroquinoléine N-substituée de formule générale:

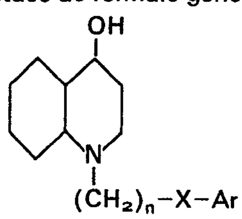

dans laquelle n, X et Ar ont la même signification que dans la revendication 1 avec un composé de formule générale:

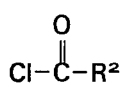

dans laquelle $R^2$ représente un atome de chlore ou un radical phénoxy pour obtenir un dérivé carbonyloxy de formule générale:

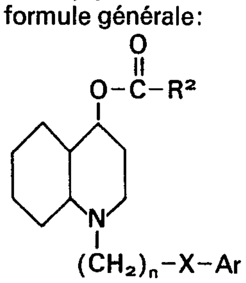

dans laquelle n, X, $R^2$ et Ar ont la même signification que précédemment, ledit dérivé est ensuite condensé avec de l'ammoniac ou une amine primaire de formule générale:

$$H_2N-R^1$$

dans laquelle $R^1$ a la même signification que précédemment, de préférence dans un milieu inerte et à la température ambiante afin de former l'ester carbamique de décahydroquinoléinol correspondant sous la forme de sa base libre, ester qui peut être traité par la suite, si on le désire, avec un acide organique ou inorganique pour obtenir un sel d'addition non toxique.

23. Procédé selon la revendication 22, caractérisé en ce que le solvant est le benzène, le toluène, le dichlorométhane, et le milieu est le toluène, le dichlorométhane ou un mélange toluène/dichlorométhane.

## Patentansprüche

1. Decahydrochinolinolderivate gemäss der allgemeinen Formel:

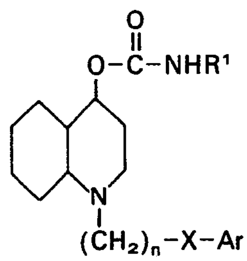

und pharmazeutisch annehmbare Säureadditionssalze derselben, in welchen

$R^1$ Wasserstoff, einen Methyl-, Ethyl-, n-Propyl-, n-Butyl- oder Phenylrest oder einen Phenylrest mit einem oder zwei Substituenten, ausgewählt aus Chlor, Brom und einer Methylgruppe, bedeutet,

Ar einen Phenyl- oder Naphtylrest oder einen Phenylrest mit einem oder zwei Substituenten, ausgewählt aus Fluor, Chlor und Brom sowie Methyl, Methoxy, Acetyl und Cyan, bedeutet,

X für Sauerstoff oder Schwefel steht, und n 2 oder 3 bedeutet.

2. Decahydrochinolinolderivate nach Anspruch 1 in Form der axialen Epimeren.

3. Decahydrochinolinolderivate nach Anspruch 1 in Form der äquatorialen Epimeren.

4. 4-Carbamoyloxy-1-[3-(4-fluorphenoxy)-propyl]transdecahydrochinolin (axiale Form) und pharmazeutisch annehmbare Säureadditionssalze desselben.

5. 1-[3-(4-Bromphenoxy)propyl]-4-carbamoyloxytransdecahydrochinolin (axiale Form) und pharmazeutisch annehmbare Säureadditionssalze desselben.

6. 4-Carbamoyloxy-1-[3-(4-cyanphenoxy)-propyl]transdecahydrochinolin (axiale Form) und pharmazeutisch annehmbare Säureadditionssalze desselben.

7. 1-[3-(4-Fluorphenoxy)propyl]-4-(N-methylcarbamoyloxy)transdecahydrochinolin (axiale Form) und pharmazeutisch annehmbare Säureadditionssalze desselben.

8. 4-(N-Ethylcarbamoyloxy)-1-[3-(4-fluorphenoxy)propyl]transdecahydrochinolin (axiale Form) und pharmazeutisch annehmbare Säureadditionssalze desselben.

9. 1-[3-(4-Fluorphenoxy)propyl]-4-(N-n-propylcarbamoyloxy)transdecahydrochinolin (axiale Form) und pharmazeutisch annehmbare Säureadditionssalze desselben.

10. 4-(N-n-Butylcarbamoyloxy)-1-[3-(4-fluorphenoxy)propyl]transdecahydrochinolin (axiale Form) und pharmazeutisch annehmbare Säureadditionssalze desselben.

11. 4-Carbamoyloxy-1-[2-(2,6-dichlorphenoxy)ethyl]transdecahydrochinolin (axiale Form) und pharmazeutisch annehmbare Säureadditionssalze desselben.

12. 4-Carbamoyloxy-1-[3-(4-fluorphenoxy)propyl]transdecahydrochinolin (äquatoriale Form) und pharmazeutisch annehmbare Säureadditionssalze desselben.

13. 4-[3-Chlor-4-methylphenyl)carbamoyloxy]-1-[3-(4-fluorphenoxy)propyl]transdecahydrochinolin (äquatoriale Form) und pharmazeutisch annehmbare Säureadditionssalze desselben.

14. 1-[2-(4-Bromphenoxyethyl]-4-carbamoyloxytransdecahydrochinolin (axiale Form) und pharmazeutisch annehmbare Säureadditionssalze desselben.

15. 4-Carbamoyloxy-1-[2-(1-naphthyloxy)-ethyl]transdecahydrochinolin (axiale Form) und pharmazeutisch annehmbare Säureadditionssalze desselben.

16. 4-Carbamoyloxy-1-[2-(2-naphthyloxy)-ethyl]transdecahydrochinolin (axiale Form) und pharmazeutisch annehmbare Säureadditionssalze desselben.

17. Decahydrochinolinolderivate gemäss einem der Ansprüche 1 bis 16, in welchen das pharmazeutisch annehmbare Säureadditionssalz das Hydrochlorid oder Methansulfonat ist.

18. Neues Arzneimittel, wobei das aktive Prinzip ein Decahydrochinolinolderivat gemäss einem der Ansprüche 1 bis 16 ist.

19. Neues Arzneimittel zur Behandlung kardialer Arrhythmieen verschiedener Genese, insbesondere Arrhythmieen aufgrund eines Myocardinfarktes, wobei das Arzneimittel ein Decahydrochinolinolderivat gemäss einem der Ansprüche 1 bis 16 ist.

20. Neues Arzneimittel zur Lokalanästhesie, wobei das Arzneimittel ein Decahydrochinolinolderivat gemäss einem der Ansprüche 1 bis 16 ist.

21. Pharmazeutisches oder veterinärmedizinisches Präparat, enthaltend als wesentlichen aktiven Bestandteil mindestens ein Dehydrochinolinolderivat gemäss einem der Ansprüche 1 bis 3 in Verbindung mit einem pharmazeutischen Träger oder Streckmittel.

22. Verfahren zur Herstellung der Decahydrochinolinolderivate gemäss Anspruch 1, dadurch gekennzeichnet, dass ein N-substituiertes 4-Hydroxytransdecahydrochinolin der allgemeinen Formel:

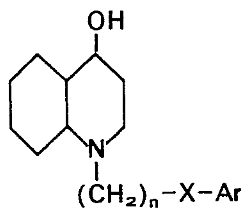

in welcher n, X und Ar die in Anspruch 1 angegebene Bedeutung haben, in einem inerten organischen Lösungsmittel und bei Zimmertemperatur mit einer Verbindung der allgemeinen Formel:

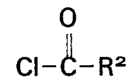

in welcher $R^2$ für ein Chloratom oder einen Phenoxyrest steht, unter Bildung eines Carbonyloxyderivates der allgemeinen Formel:

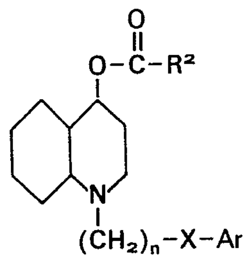

in welcher n, X, $R^2$ und Ar die obige Bedeutung haben, umgesetzt wird, das anschliessend mit Ammoniak oder einem primären Amin der allgemeinen Formel:

$$H_2N-R^1$$

in welcher $R^1$ die obige Bedeutung hat, vorzugsweise in einem inerten Medium und bei Zimmertemperatur zur Bildung des entsprechenden Carbaminsäureesters des Decahydrochinolinols in Form einer freien Base kondensiert wird, die dann gegebenenfalls mit einer organischen oder anorganischen Säure zur Bildung eines pharmazeutisch annehmbaren Säureadditionssalzes behandelt wird.

23. Verfahren gemäss Anspruch 22, dadurch gekennzeichnet, dass das Lösungsmittel Benzol, Toluol oder Dichlormethan und das Medium Toluol, Dichlormethan oder eine Toluol/Dichlormethanmischung ist.

## Claims

1. Decahydroquinolinol derivatives corresponding to the general formula:

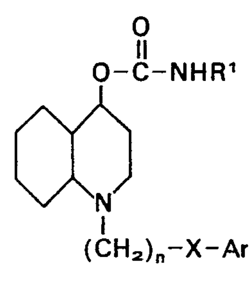

and pharmaceutically acceptable acid addition salts thereof in which

$R^1$ represents hydrogen, a methyl, ethyl, n-propyl, n-butyl or phenyl radical or a phenyl radical having one substituent or two substituents selected from chlorine, bromine atoms and a methyl group,

Ar represents a phenyl or naphthyl radical or a phenyl radical having one substituent or two substituents selected from fluorine, chlorine and

bromine atoms and methyl, methoxy, acetyl and cyano groups,

X represents oxygen or sulphur, and

n represents 2 or 3.

2. Decahydroquinolinol derivatives according to claim 1 in the form of axial epimers.

3. Decahydroquinolinol derivatives according to claim 1 in the form of equatorial epimers.

4. 4-Carbamoyloxy-1-[3-(4-fluorophenoxy)-propyl]transdecahydroquinoline (axial form) and pharmaceutically acceptable acid addition salts thereof.

5. 1-[3-(4-Bromophenoxy)propyl]-4-carbamoyloxytransdecahydroquinoline (axial form) and pharmaceutically acceptable acid addition salts thereof.

6. 4-Carbamoyloxy-1-[3-(4-cyanophenoxy)-propyl]transdecahydroquinoline (axial form) and pharmaceutically acceptable acid addition salts thereof.

7. 1-[3-(4-Fluorophenoxy)propyl]-4-(N-methylcarbamoyloxy)transdecahydroquinoline (axial form) and pharmaceutically acceptable acid addition salts thereof.

8. 4-(N-Ethylcarbamoyloxy)-1-[3-(4-fluorophenoxy)propyl]transdecahydroquinoline (axial form) and pharmaceutically acceptable acid addition salts thereof.

9. 1-[3-(4-Fluorophenoxy)propyl]-4-(N-n-propylcarbamoyloxy)transdecahydroquinoline (axial form) and pharmaceutically acceptable acid addition salts thereof.

10. 4-(N-n-Butylcarbamoyloxy)-1-[3-(4-fluorophenoxy)propyl]transdecahydroquinoline (axial form) and pharmaceutically acceptable acid addition salts thereof.

11. 4-Carbamoyloxy-1-[2-(2,6-dichlorophenoxy)ethyl]transdecahydroquinoline (axial form) and pharmaceutically acceptable acid addition salts thereof.

12. 4-Carbamoyloxy-1-[3-(4-fluorophenoxy)-propyl]transdecahydroquinoline (equatorial form) and pharmaceutically acceptable acid addition salts thereof.

13. 4-[(3-Chloro-4-methylphenyl)carbamoyloxy]-1-[3-(4-fluorophenoxy)propyl]transdecahydroquinoline (equatorial form) and pharmaceutically acceptable acid addition salts thereof.

14. 1-[2-(4-Bromophenoxy)ethyl]-4-carbamoyloxytransdecahydroquinoline (axial form) and pharmaceutically acceptable acid addition salts thereof.

15. 4-Carbamoyloxy-1-[2-(1-naphthyloxy)-ethyl]transdecahydroquinoline (axial form) and pharmaceutically acceptable acid addition salts thereof.

16. 4-Carbamoyloxy-1-[2-(2-naphthyloxy)-ethyl]transdecahydroquinoline (axial form) and pharmaceutically acceptable acid addition salts thereof.

17. Decahydroquinolinol derivates according to any of claims 1 to 16 wherein the pharmaceutically acceptable acid addition salt is the hydrochloride or the methanesulphonate.

18. Novel medicament whereby the active principle is a decahydroquinolinol derivative according to any of claims 1 to 16.

19. Novel medicament useful in the treatment of cardiac arrhythmias of various origins, in particular of arrhythmias due to myocardial infarction, whereby the said medicament is a decahydroquinolinol derivative according to any of claims 1 to 16.

20. Novel medicament useful for inducing local anaesthesia whereby the said medicament is a decahydroquinolinol derivative according to any of claims 1 to 16.

21. A pharmaceutical or veterinary composition comprising, as essential active ingredient, at least one decahydroquinolinol derivative according to any of claims 1 to 3, in association with a pharmaceutical carrier or excipient.

22. Process for preparing decahydroquinolinol derivatives according to claim 1 whereby a N-substituted 4-hydroxytransdecahydroquinoline of general formula:

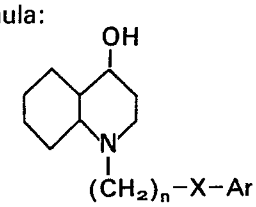

in which

n, X and Ar have the same meaning as in claim 1, is reacted, in an inert organic solvent and at room temperature, with a compound of general formula:

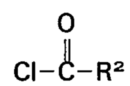

in which

$R^2$ represents a chlorine atom or a phenoxy radical to obtain a carbonyloxy derivative of general formula:

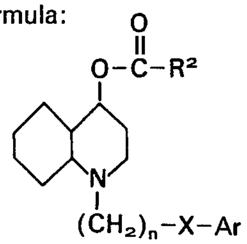

in which

n, X, $R^2$ and Ar have the same meaning as above, which is subsequently condensed with ammonia or a primary amine of general formula:

$$H_2N-R^1$$

in which

$R^1$ has the same meaning as above, preferably in an inert medium and at room temperature to form the corresponding carbamic ester of decahydroquinolinol in free base form which is then treated, if desired, with an organic or inorganic acid to provide a pharmaceutically acceptable acid addition salt.

23. Process according to claim 22 whereby the solvent is benzene, toluene, dichloromethane and the medium is toluene, dichloromethane or a toluene/dichloromethane mixture.